# EUROPEAN PATENT APPLICATION

(11) **EP 3 666 298 A1**
(43) Date of publication of application: **17.06.2020**
(21) Application number: 18212299.4
(22) Date of filing: 13.12.2018
(51) Int. Cl.: A61L 27/24, A61K 38/39, A61K 9/00, C12N 5/07, A61K 8/65

(54) **A COLLAGEN FORMULATION SUITABLE FOR INJECTION**

(71) Applicant: Global Stem Cell Technology, 9940 Evergem (BE)
(72) Inventor: SPAAS, Jan, 9940 Evergem (BE); Van Hecke, Lore, 9940 Evergem (BE); Depuydt, Eva, 9940 Evergem (BE); BROECKX, Sarah, 9940 Evergem (BE)
(74) Representative: Brantsandpatents bvba

(57) **Abstract**

The current invention relates to a method for preparing a collagen formulation suitable for injection, comprising the steps: a) providing a collagen solution having a pH which is lower than 6; and b) adding an amount of liquid neutralizing agent to said collagen solution, resulting in the collagen formulation wherein said collagen formulation has a pH of between 6.5 and 8.5. The inventions also relates to a kit for preparing said collagen formulation and the kit and the collagen formulation for use in the treatment of a pathology in the musculoskeletal system in a subject.

## Description

### TECHNICAL FIELD

The present invention relates to the preparation of a collagen formulation suitable for injection, and particularly for injection into a mammal for the therapeutic treatment of pathological conditions.

### BACKGROUND

Damage to joints, tendons or ligaments can arise from overuse, trauma, diseases, and as a result of the aging process. To date, most applied therapies include conservative treatments such as administration of NSAIDs, corticoid treatment, surgical treatment, bandaging, etc. These treatments reveal a high symptom recurrence rate, but only an equivocal efficacy rate. The injections of NAIDS and corticoids also carry the risk of a tendon or ligament rupture, an infection, a skin depigmentation and a subdermal atrophy. The surgical measures include debridement and repair of the associated pathologic tissue. However, invasive or minimal invasive surgery has many potential complications such as deep infection, damage to neurovascular structures, and scar formation. Surgery is also expensive and carries the additional risks associated with regional or general anesthesia. Moreover, these treatments are often found to be ineffective and time-consuming.

Collagen has been used commercially and clinically for some time to replace or augment hard or soft connective tissue, such as skin, tendons, cartilage, bone and interstitium. Solid and injectable collagen formulations are known in the art.

EP 297 609 7 discloses a composition comprising crosslinked collagen, platelet rich plasma and an inorganic salt. Collagen is used as an activator of PRP in order to form clotting of PRP, whereby the collagen is formulated so as to control the rate of release of growth factors from the clot. Said composition is injected in the affected tissue using a needle and/or a syringe, and a gelling and/or hardening agent is, if necessary, added *in situ.* One or more needles are configured to deliver said composition and agent simultaneously, or substantially simultaneously, to the affected tissue. In some cases even separate needles are used to deliver the components to the tissue at the same time or one after the other.

Injection of collagen can encounter difficulties as it is typically formulated in liquid form. However, the delivery and residence of such injected formulation in the injury site remains a challenge due to its liquid state and therefore the potential loss of material to the surrounding tissue.

There thus remains a need in the art for an improved collagen formulation with further physical properties to expand the selection of formulations available to practitioners of skill in the art. Furthermore, there is a need for safe and effective treatment which can be used on a long-term basis without side effects and which also promotes rebuilding of the injured/diseased connective tissues.

The present invention aims to resolve at least some of the problems and disadvantages mentioned above.

### SUMMARY OF THE INVENTION

The present invention and embodiments thereof serve to provide as a solution to one or more of above-mentioned disadvantages. To this end, the present invention relates to a method for preparing a collagen formulation suitable for injection according to claim 1.

The method of preparation is straightforward and results in a liquid and flowable collagen formulation, which can easily be injected in the affected tissue. Said collagen formulation has, amongst others, the intrinsic capability of gelling. The transition of the liquid state to the solid state positions the components of the collagen formulation at the site of injury aiding the repair and regeneration of the affected tissue.

Preferred embodiments of the method are shown in any of the claims 1 to 13.

The current invention furthermore relates to a kit for the preparation of a collagen formulation suitable for injection according to claim 14.

The kit of the current invention provides for all the components needed to prepare a collagen formulation, in that way that a safe and effective formulation can be prepared for injection.

A preferred embodiment of the kit is shown in claim 15.

In a final aspect of current invention the kit and collagen formulation are used in a treatment according to claim 16.

The kit and the collagen formulation are especially developed for treatment of pathologies in the musculoskeletal system in animals. A non-invasive treatment contributing to a quick recovery and restored performance.

The method and kit provide for a collagen formulation, wherein the collagen formulation in the kit is prepared according to the method of the current invention, and wherein said collagen formulation has the capacity to form a stable gel once injected, having an increased cell retention, cell ingrowth, matrix elasticity and shock absorbance.

### DESCRIPTION OF THE FIGURES

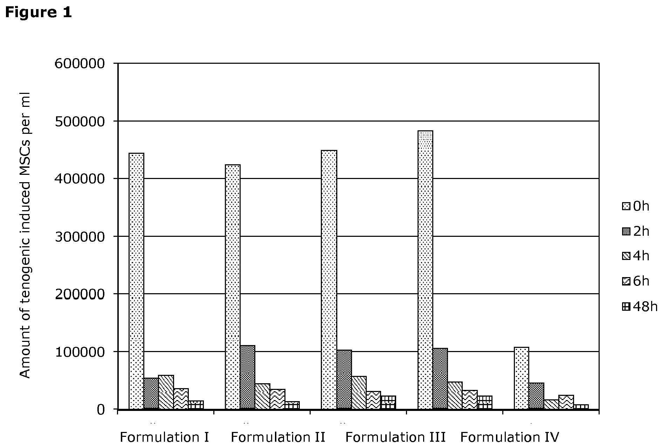

**Figure 1** shows a graphical representation of the impact of collagen and its ratio on cell retention in a perfusion culture system.

### DETAILED DESCRIPTION OF THE INVENTION

The present invention concerns a method and a kit for preparing a collagen formulation suitable for injection.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

Whereas the terms "one or more" or "at least one", such as one or more or at least one member(s) of a group of members, is clear *per se*, by means of further exemplification, the term encompasses *inter alia* a reference to any one of said members, or to any two or more of said members, such as, *e*.*g*., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The terms "patient", "subject", "animal", or "mammal" are used interchangeably and refer to a mammalian subject to be treated, with equine patients being preferred.

The terms "plasma" or "blood plasma" as used herein refers to the liquid component of blood holding the blood cells in blood in suspension. Blood plasma comprises water, dissolved proteins like serum albumins, globulins and fibrinogen, glucose, clotting factors, electrolytes, hormones, carbon dioxide and oxygen.

The terms "platelet rich plasma" or "PRP" refers to a plasma which has a concentration of platelets greater than the concentration of platelets in peripheral blood.

The term "collagen" as used herein, refers to a polypeptide containing a repeating Gly-X-Y triplet, where Gly is glycine and where X and Y can be any amino acid but are frequently the amino acids proline and hydroxyproline.

The term "telocollagen" in current invention refers to collagen molecules that lack both the N- and C-terminal propeptides typically comprised in procollagen but still contain the telopeptides. The telopeptides of fibrillar collagen are the remnants of the N-and C-terminal propeptides following digestion with native N- and C-proteinases.

The term "atelocollagen" refers to collagen molecules lacking both the N- and C terminal propeptides typically comprised in procollagen, but including a sufficient portion of its telopeptides such that under suitable conditions it is capable of forming fibrils.

The term "derivatized collagen" in present invention refers to chemically modified collagen. Examples of derivatized collagen include, but are not limited to, deamidated, methylated, succinylated, and phosphorylated collagen.

The term "fibrillogenesis" as used herein refers to the precipitation of soluble collagen in the form of fibrils.

The term "musculoskeletal system" includes the muscular and skeletal system. The musculoskeletal system comprises bones, cartilage, muscles, ligaments, and tendons. Primary functions of the musculoskeletal system include support of the body, provision of motion, and protection of vital organs.

In a first aspect, the invention provides a method for preparing a collagen formulation suitable for injection, comprising the steps: a) providing a collagen solution having a pH which is lower than 6, and b) adding an amount of liquid neutralizing agent to the initial collagen solution, resulting in the collagen formulation wherein said collagen formulation has a pH of between 6.5 and 8.5.

Hereafter the term "collagen solution", "initial collagen solution" or "acidic collagen solution" refers to the liquid solution of collagen having a pH lower than 6 and not suitable for injection.

Collagen is a proteinaceous material comprising the major fibrous element of body including skin, bone, tendon, cartilage, blood vessels, and teeth. Its biological purpose is to hold cells together in discrete units; and secondarily it has a directive role in developing tissues. The collagen proteins are distinctive in their physical characteristics in that they form insoluble fibers possessing high tensile strength. It is the fibrous nature of the collagen that serves to hold the various body structures and components together.

While the basic molecular structure of collagen may be modified to meet the needs of particular tissues, all collagens are organized into a common structure consisting of three polypeptide chains that form a triple stranded helix. These triple stranded helical units, in turn, are formed into a quarter-staggered array of linearly aligned bundles which make up collagen fibers. The collagen fibers are stabilized by covalent cross-links.

Collagen is secured from mammalian sources whereby extraneous proteinaceous material is removed by various dissolution, precipitation and filtration techniques to leave a pure collagenous product. In a preferred embodiment the collagen is obtained from mammalians of the Bovidae or Equidae family, more preferably the collagen is of bovine or equine origin. Unfortunately this pure natural collagen may induce antigenic response in the host subject. Such response is generated by the end portions of the collagen fibrils which are not helically bound. Fortunately these end portions of collagen can be cleaved therefrom by treatment with a proteolytic enzyme, e.g. pepsin. After digestion with pepsin, the cleaved peptide ends are discarded and only the central collagen bundles (tropocollagen) remain. These central collagen bundles have greatly reduced antigenicity and they can be used for reconstructive surgery without undue antigenic side effects.

Although reduced antigenic collagen is preferred, non-cleaved collagen that has been isolated from mammalian sources may also be used. It is only necessary that the collagen is prepared in a sterile condition in an aqueous solution. Some inclusion of materials commonly associated with the collagen, e.g., polysaccharides, can be tolerated and do not interfere with the benefits of the injury healing.

The methodology of the current invention is particularly useful for treating subjects, such as animals on location. At a pH lower than 6, the collagen will be in a liquid state and thus easy transportable. Upon increasing the pH, the collagen will transition from a liquid state to a semi-solid and solid state through jellification. When injected prior to or at the onset of this transition, it was shown that the collagen performed significantly better. The methodology is furthermore straightforward, as only a minimal amount of steps are necessary to obtain said formulation and can easily be performed on the site where the injection will take place. The method further limits the risk of human error and empowering a safe and effective formulation, which is of great benefit for patients, and in general healthcare. It was furthermore seen that the invention or any aspect thereof leads to an improvement in the field of injury healing and tissue regeneration, which on its turn contributes to performance and wellbeing.

Additionally, it turned out in the development work that collagen materials with a pH between 6.5 and 8.5, preferably 7 and 7.8, improved the compatibility in the affected tissue in such a manner that physiological conditions corresponding to the proper healing course were to be found in the histological evaluation of tissue samples taken from the implantation site.

Said collagen may be human collagen.

According to another or further embodiment, the collagen is a type I or biologically active fragment therefrom. Type I collagen has a native involvement in the intrinsic clotting cascade and is an alternative to thrombin for platelet activation. Type I collagen is the major protein component in mammalian connective tissue and is commonly used as a natural scaffold for regenerative tissue engineering. An excess of collagen to the liquid neutralizing agent results in a better gel formation once administered in the affected tissue.

Said collagen solution may comprise tropocollagen, telocollagen, atelocollagen, derivatized collagen, or a combination thereof.

In another or further embodiment the collagen solution comprises a sufficient portion of its telopeptides such that under suitable conditions it is capable of forming fibrils.

According to another or further embodiment, the collagen solution comprises fibrillated collagen. In a further embodiment, the collagen solution comprises a recombinant collagen. In another or further embodiment, the collagen solution comprises recombinant collagen generated in a plant.

The provided collagen solution has an acidic pH lower than 6. Preferably the collagen solution has an acidic pH lower than 5, more preferably between pH 1.5 and 4.5, more preferably between pH 1.5 and 3.5, more preferably between pH 1.5 and 2.5, even more preferably pH 2.

The collagen may be present in an acid solution at a concentration between 5.2 to 8.4 mg/ml. According to a particular embodiment, the collagen may be present in the acid solution at a concentration of about 5.4 and 8.3 mg/ml. As mentioned, the collagen is soluble in an acidic solution and has a denaturized state, which enables safe and stable storage of the collagen solution at controlled conditions. Such a collagen solution may be stored in liquid nitrogen to prevent degradation. The collagen solution can be lyophilized for ease of storage and transportation. An exemplary concentration of HCl used to solubilize collagen is about 10 mM HCl. A concentration of acetic acid which may be used to solubilize collagen is about 5 mM acetic acid.

According to a particular embodiment, the collagen is present in the solution at a concentration between 4 and 9 mg/ml, more preferably between 5 and 8.5 mg/ml. Low concentrations of collagen are preferred for an efficient aggregation of the soluble collagen to form fibril bundles that resist stretch and tension forces.

Following, the collagen solution may be altered to promote fibrillogenesis thereof. Fibrillogenesis may be performed in a variety of ways including neutralization of the pH, increasing the temperature and/or the ionic strength.

Neutralizing the pH of the acidic collagen solution to a pH between 6.5 and 8.5, makes the collagen formulation suitable for injection, as this pH mimics the physiological conditions of the tissue to be treated. A normal pH of interstitial fluids and connective tissue is 7.34 and 7.4. In a preferred embodiment of the invention, the pH of the collagen formulation suitable for injection is between 7 and 7.8, more preferably between 7.2 and 7.6, most preferably 7.4.

When the collagen solution is combined with said liquid neutralizing agent, the neutralized pH enables the formation of a stable triple-helical structure of the collagen and the collagen formulation safe for use and suitable for injection. Said collagen formulation is liquid and thus flowable for a given period of time, depending on the amount of added liquid neutralizing agent, at room temperature (20 - 28 °C). The collagen then undergoes a phase transition to create a stable gel. The gel forms at room temperature and more rapidly at mammalian body temperature, that is, approximately 37 °C. Accordingly, the collagen formulation offers functional properties as an injury dressing, due to its capacity to form a gel once injected, having an increased cell retention, cell ingrowth, matrix elasticity and shock absorbance.

In a preferred embodiment the liquid neutralizing agent is selected from the group consisting of a plasma, a physiological solution and/or an alkaline solution.

The plasma, physiological solution and alkaline solution change the pH of the acidic collagen solution to a neutral pH, wherein said neutral pH is between 6.5 and 8.5, preferably between 7 and 7.8, more preferably between 7.2 and 7.6, e.g. 7.35.

The combination of plasma and an alkaline solution, the combination of plasma and a physiological solution or the combination of an alkaline solution and a physiological solution can be added to the initial collagen solution to neutralize the pH of said solution to obtain the collagen formulation having a pH between 6.5 and 8.5.

The physiological solutions in the present invention are solutions with a physiological pH, wherein said physiological pH is between 7.35 and 7.45, preferably pH 7.4. A balanced isotonic salt solution with a suitable pH and concentration can be added to the collagen solution. The salt solution may comprise or may be an inorganic salt solution of alkali or alkaline earth metal, like calcium, magnesium, sodium bicarbonate, sodium phosphate. The inorganic salt may even be a sodium salt, chloride salt, potassium salt, calcium salt, magnesium salt, phosphate salt, sulfate salt or a carboxylate salt. Preferably, salts with plurivalent cations (e.g. CaCl2) are avoided, as they may act as flocculants. In order to generate the collagen formulation suitable for injection described herein a mixture of the collagen solution and salt solution may be prepared.

Also blood serum, as a physiological solution, could be added to the collagen solution to attain the physiological pH conditions. Sera of different animal sources can be obtained like, among others, bovine serum, calf serum, horse serum, newborn calf serum, fetal bovine serum.

Alkaline solutions like NaOH or another metal hydroxide can be used as well to neutralize the pH of an acidic solution. Another exemplary alkaline solution that may be added to neutralize the pH of the collagen is Na₂HPO₄ (pH 11.2). Typically, an amount of liquid neutralizing agent is calculated such that the final pH of the collagen is about 6.5 and 8.5, e.g. 7.38.

Said liquid neutralizing agent may be plasma, preferably a platelet rich plasma (PRP).

Collagen and plasma are naturally occurring substances having intrinsic biological properties for coagulation, wherein the coagulation involves the activation of platelets mediated by the collagen, particularly type I collagen.

When plasma is being used to neutralize the collagen, the plasma may be isolated from the blood of mammals, more preferably from human blood or from mammals of the Bovidae or Equidae family. The plasma can be of human, bovine or equine origin. The plasma could also be any synthetic plasma, mimicking the biological blood plasma. Normally blood plasma is controlled at pH 7.4, with a small range between pH 7.35 and 7.45. Plasma has a buffering capacity on its own by its intracellular proteins, including hemoglobin. Protein molecules, in general, possess basic and acidic groups which act as H⁺ acceptors or donors respectively if H⁺ is added or removed.

Platelet rich plasma contains more platelets than peripheral blood. While normal platelet counts may range from about 150,000 to about 450,000 per microliter in humans. Normal platelet counts ranges in horses from 75,000 to 300,000; in ruminants from 250,000 to 730,000; and even up to 1,000,000 in calves. Depending on the mammalian species the normal platelet count is highly variable. Platelet concentrations of PRP may be in the range of about 300,000 to about 9,000,000 per microliter or more of blood. Platelet rich plasma is formed from blood, and is obtained using autologous, allogenic, or pooled sources of platelets and/or plasma.

PRP is formed from a variety of mammalian sources, preferably from human blood or from mammals of the Bovidae or Equidae family. The plasma can be of human, bovine or equine origin.

Platelet rich plasma is blood plasma that has been enriched with platelets. Typically, platelet rich plasma contains 95 % platelets, 4 % red blood cells and 1 % white blood cells. As a concentrated source of autologous platelets, platelet rich plasma contains (and releases through degranulation) several different growth factors and other cytokines that stimulate healing of bone and soft tissue. Platelet rich plasma functions as a tissue sealant and drug delivery system, with the platelets initiating injury repair by releasing locally acting growth factors via alpha granules degranulation. The secretory proteins contained in the alpha granules of platelets include platelet-derived growth factor (PDGF-AA, BB, and AB isomers), transforming growth factor-β (TGF-β), platelet factor 4 (PF4), interleukin-1 (IL-1), platelet-derived angiogenesis factor (PDAF), vascular endothelial growth factor (VEGF), epidermal growth factor (EGF), platelet-derived endothelial growth factor (PDEGF), epithelial cell growth factor (ECGF), insulin-like growth factor (IGF), osteocalcin (Oc), osteonectin (On), fibrinogen (Ff), vitronectin (Vn), fibronectin (Fn), and thrombospondin-1 (TSP-1). These growth factors aid healing by attracting undifferentiated cells in the newly formed matrix and triggering cell division. Platelets in PRP also play a role in host defense mechanisms at the wound site by producing signaling proteins that attract macrophages. PRP may also contain a small number of leukocytes that synthesize interleukins as part of a non-specific immune response. And PRP may suppress cytokine release and limit inflammation, interacting with macrophages to improve tissue healing and regeneration, promote new capillary growth, and accelerate epithelialization in chronic wounds.

Injection of the collagen formulation comprising collagen and platelet rich plasma improves in pain relief and functioning of the treated tissue. The use of platelet rich plasma is safe and less invasive.

The ratio of the collagen solution and liquid neutralizing agent in current invention is optimal for a stable collagen gel in physiological conditions *in vitro* and *in situ,* which is *in situ* associated with an improved gel formation at the site of administration.

A ratio with an excess of the collagen solution to the liquid neutralizing agent is preferred. In a preferred embodiment the ratio of the collagen solution and the liquid neutralizing agent is between 4:1 to 1:1. A more preferred ratio of the collagen solution and liquid neutralizing agent is between 3:1 and 1:1, more preferably between 11:4 and 5:4, more preferably between 5:2 and 3:2, more preferably between 9:4 and 7:4. In a more preferred embodiment the ratio of the collagen solution and the liquid neutralizing agent is 2:1.

A ratio with an excess of the collagen solution to plasma is preferred. In a particularly preferred embodiment of present invention the ratio of the collagen solution and plasma is between 4:1 and 1:1. A more preferred ratio of the collagen solution and plasma is between 3:1 and 1:1, more preferably between 11:4 and 5:4, more preferably between 5:2 and 3:2, more preferably between 9:4 and 7:4. In an even more preferred embodiment of present invention the ratio of the collagen solution and plasma is 2:1. In another or further embodiment the ratio of the collagen solution and platelet rich plasma in present invention is preferably between 4:1 and 1:1, more preferably between 3:1 and 1:1, more preferably between 11:4 and 5:4, more preferably between 5:2 and 3:2, more preferably between 9:4 and 7:4, and most preferably 2:1. Overall, a ratio with an excess the collagen solution to platelet rich plasma is preferred.

In a further or other embodiment, stem or progenitor cells are added to the collagen formulation suitable for injection.

As used herein, the terms "stem cells" or "progenitor cells" refer to those cells which are multi-, toti- or pluripotent, have self-renewal capacities and are capable to give rise to multiple cell types or cell lineages.

Stem cells possess the property of differentiating into different cell types, can multiply massively, migrate spontaneously to damaged tissues, produce important factors for tissue repair and possess immune-modulating properties. A collagen formulation suitable for injection comprising collagen, platelet rich plasma or plasma and stem cells is desirable to increase the effectiveness of said formulation or create a synergistic effect. Plasma stimulates the stem cells after implantation. Preferably, both the stem cells and plasma will come from the same donors for reasons of compatibility. Carrier substances, like collagen, are used to combat gravity: stem cells follow the law of gravity and can therefore sometimes reach higher injuries difficult without a carrier in which they can migrate. Moreover, the carriers themselves can also have a beneficial effect on a pathological environment in which they, on the one hand, restore the tissue and, on the other hand, form a good stem cell niche and help to complete the differentiation on site.

In a preferred embodiment, said stem cells are mesenchymal stem cells. Said mesenchymal stem cells or MSCs may be derived from blood, bone marrow, adipose tissue, or muscle. In a further preferred embodiment, said mesenchymal stem cells or MSCs are peripheral blood MSCs. Said MSCs are obtainable by any method known in the art, for instance via the method as described in WO2014/053418.

Various sources of mesenchymal stem cells have been described in horses and other mammals; mainly mesenchymal stem cells from bone marrow, adipose tissue, umbilical cord matrix and umbilical cord blood have been described. However, isolating mesenchymal stem cells from above mentioned sources is highly invasive.

Preferably, the mesenchymal stem cells of the present invention are isolated from mammalian blood, more preferably from peripheral blood. In particular, blood is used from mammalians, most preferably from horse. Blood is not only a non-invasive and painless source, but also safe and easy to collect and therefore easily accessible.

In an embodiment of the current invention, the collagen formulation suitable for injection comprises mesenchymal stem cells induced towards tenocytes or chondrocytes; such methods being described in WO WO2014 053 418, WO 2014 053 420 and WO 2015 082 014.

In a possible embodiment, blood is used from a donor which will later also be recipient of the isolated stem cells, preferably MSCs. In another embodiment, blood is used from donors, the donors preferably of the same family, sex or race as the recipient of the stem cells, isolated from the blood of the donors. In particular, when donor blood is used, these donors will be tested for current diseases or pathologies in order to prevent the risk of horizontal transmission of these pathologies or diseases via the stem cells.

In a preferred embodiment the stem cells and plasma originate from the same donor, to prevent immune compatibility problems. In a more preferred embodiment the stem cells and platelet rich plasma originate from the same donor, to prevent immune compatibility problems.

In another or further embodiment of current invention the stem cells are induced or differentiated cells derived from stem cells isolated from the mammalian blood, preferably peripheral blood.

In a preferred embodiment of the present invention the ratio of the stem cells (e.g. MSCs) and the collagen solution is between 1:4 and 1:1.

Preferably the collagen is provided in an excess to the stem cells. A favorable ratio of the stem cells and the collagen solution is 1:3, more preferably 1:2. In a particular embodiment the collagen formulation in current invention comprises collagen, plasma and stem cells, wherein the collagen is provided in excess and the ratio of the plasma and stem cells is 1:1. As an exemplary the collagen, plasma and stem cells have a ratio of 1:0.5:0.5. In another exemplary the collagen, plasma and stem cells (e.g. MSCs) have a ratio of 2:0.5:0.5.

In a particularly preferred embodiment the pH of said collagen solution in step a) is adjusted to a pH between 4.5 and 5.5 with an alkaline solution, prior to adding said liquid neutralizing agent.

A ratio between 4:1 and 1:1 is preferred for the collagen solution and the alkaline solution, more preferably between 3:1 and 1:1, even more preferred between 2:1 and 1:1.

Following to the addition of the liquid neutralizing agent to the collagen solution, the resulting collagen mixture may preferably be cooled between 0 °C and 7 °C for a period of at least six months. The minimal storage at controlled temperature minimizes issues in the work field of injecting an ineffective and cardiotoxic collagen solution as to its acidity. Additionally, the storage at controlled temperature stabilizes the collagen mixture, as collagen is sensitive for dissociation at a pH of about 5 at room temperature.

In another or further embodiment the final concentration of the collagen in said formulation suitable for injection is in the range of 3 to 8 mg/ml.

Preferably the concentration of collagen in the collagen formulation is in the range of 3 to 7.5 mg/ml, more preferably between 3.5 and 6.5 mg/ml, more preferably between 4 and 6 mg/ml, more preferably between 4.5 and 5.5 mg/ml. Also for the collagen formulation with or without stem cells the concentration of collagen is preferred in the range of 3 to 8 mg/ml.

The obtained collagen formulation with or without stem cells, preferably mesenchymal stem cells, is particularly useful for injection in a subject, such as a mammal.

In a preferred embodiment a minimal dose of 1 ml of the liquid collagen formulation is injected in the affected tissue of the subject. Particularly, a dose of 2 µl/kg of the liquid collagen formulation is injected in the affected tissue of the subject. As the body weight of subjects is highly variable it is appropriate to provide a weight based dose. Although a weight based dose is preferred, at least 1 ml of the liquid collagen formulation should be injected in the affected tissue of the subject to reveal clinical results.

Said collagen formulation is administered in an amount sufficient to cure or at least partially arrest the disease and its complications. An amount adequate to accomplish this is defined as a therapeutically effective dose. Amounts effective for this use depend on the severity of the pathology and the general state of the patient's health.

The formulation is to be administered as a liquid solution. In certain embodiments, the rheological properties of the collagen formulation of the invention are advantageous. The pH of the solution should be in the range of pH 5 to 9.5, preferably pH 6.5 to 7.5.

In another or preferred embodiment the collagen formulation suitable for injection is administered by an intravenous, intraarticular, intramuscular, or intralesional injection.

These modes of administration will strongly depend on the site of injury, being a joint, ligament, tendon or tendon sheet. In one embodiment the collagen formulation is especially for joint disorders and tendon injuries, comprising collagen type I, platelet rich plasma and stem cells. In a preferred embodiment, the collagen formulation is especially for joint disorders and tendon injuries, comprising collagen type I, platelet rich plasma and mesenchymal stem cells. The site of delivery of the collagen formulation is typically at or near the site of tissue damage. The site of tissue damage is determined by well-established methods including imaging studies. The preferred imaging study used may be determined based on the tissue type. Commonly used imaging methods include, but are not limited to MRI, X-ray, CT scan, Positron Emission tomography (PET), Single Photon Emission Computed Tomography (SPECT), Electrical Impedance Tomography (EIT), Electrical Source Imaging (ESI), Magnetic Source Imaging (MSI), laser optical imaging and ultrasound techniques.

In order to avoid damage to the collagen formulation, the cell diameter of the injection needle may be chosen accordingly. A 23G injection needle (inner diameter 0.33 mm) is a preferred injection needle as to a 25G injection needle (inside diameter 0.26 mm) because of its greater inner diameter size.

The formulation described herein can be administered to a subject in conjunction with other therapies, such as treatments for pain relief like the administration of NSAIDs or corticosteroids.

In a preferred embodiment the time point of administration is within 120 minutes after said collagen formulation suitable for injection is prepared at room temperature.

Preferably the collagen formulation is administered within 100 minutes, after said formulation is prepared at room temperature, more preferably within 80 minutes, more preferably within 60 minutes, more preferably within 40 minutes, even more preferably within 30 minutes, even more preferably within 20 minutes, even more preferably within 10 minutes, even more preferably within 8 minutes, even more preferably within 6 minutes, most preferably within 4 minutes.

As previously mentioned the transition of the liquid collagen formulation to the solidified or gelled collagen formulation depends on the added amount of liquid neutralizing agent added to the collagen solution and the temperature of the environment. Respecting the ratios as given in current invention the person skilled in the art has an hour or two for the administration of the collagen formulation at room temperature before said formulation solidifies and cannot be injected. Hereby the person skilled in the art has a sufficient amount of time to prepare the collagen formulation and safely administer said formulation. Once the collagen formulation is administered at the site of the affected tissue in the subject with a body temperature of about 37 °C, the raise in temperature aids the gelling of said formulation. Hereby the collagen formulation can disperse in the affected tissue and subsequently occupy the affected tissue assisted by the solidified collagen and repair the affected tissue. As an example the collagen formulation comprising collagen, plasma and chondrocytes in a ratio of 2:1:1 coagulates after 57 minutes at room temperature and after 18 minutes *in situ,* considering the *in situ* temperature at 37 °C. The collagen formulation comprising collagen and plasma in a 2:1 ratio coagulated after 94 minutes at room temperature and after 6 minutes *in situ.*

In a second aspect, the invention provides a kit for preparing a collagen formulation suitable for injection comprising a collagen formulation, preferably type I collagen, at a pH below 6 and at least one neutralizing agent and optionally stem cells.

The kit comprises vials or other containers suitable for biological liquids like the collagen solution, the neutralizing agent, plasma and stem cells. Preferably the stem cells are mesenchymal stem cells. The components to obtain the improved collagen formulation of current invention are provided in a package convenient for distribution to a practitioner of skill in the art. The kit further comprises means for administering the collagen formulation of the invention to the patient. The means can be any means for administering a collagen formulation known to those of skill in the art such as a syringe, a syringe and needle, a canula, etc. Preferably the syringe is a luer lock syringe. In certain embodiments, the means is a prefilled multicompartment syringe comprising the acidic collagen solution, plasma and stem cells with respect to the preferred 2:1:1 ratio.

In another or further embodiment the volume ratio of the collagen solution, the liquid neutralizing agent and the stem cells in the kit is 2:1:1.

Preferably the collagen solution and liquid neutralizing agent are added together, thereby obtaining a collagen formulation of a pH 6.5 and 8.5, e.g. 7.4, as described in the invention, and injecting said formulation in a patient at the site of injury via a method known by a person skilled in the art. Preferably the collagen formulation suitable for injection is prepared according to the embodiments of present invention, obtaining the optimal gel formation and tissue repair. In a preferred embodiment of current invention the volume ratio of the collagen solution, the liquid neutralizing agent and the mesenchymal stem cells in the kit is 2:1:1.

In a final aspect, the invention provides a kit as described in current invention and a collagen formulation with a pH between 6.5 and 8.5 for use in the treatment of a pathology in the musculoskeletal system in a subject.

Disorders of the musculoskeletal system most often involve motion deficits, functional disorders, and lameness. The degree of impairment depends on the specific problem and its severity. In horses and dogs, musculoskeletal injuries are a major source of debilitating pain, economic loss, and loss of athleticism. Degenerative joint disease is much more common and has a greater economic importance than acute traumatic injuries in performance horses. In addition, tendon injury is a common debilitating injury in performance horses. The healing response is prolonged, and the resultant repair tissue is usually of inferior mechanical strength. Consequently, the prognosis for return to previous levels of performance is poor.

According to a preferred embodiment of the invention, the subject has a disorder of the musculoskeletal system, preferably the joint, cartilage, ligament, tendon, tendon sheath or a combination of different connective tissues. Ligaments are bands of tissue that connect bones. Tendons are bands of tissue that connect muscle to bone. The tendon sheath is the tissue that surrounds and lubricates the tendon. Injury to any of these soft tissues can produce inflammation, pain, and stiffness and cause considerable discomfort and reduce the state of wellbeing and performance.

Pathologies in the musculoskeletal system in present invention are, but not limited to desmitis, tendinitis, synovitis and (osteo)arthritis. These disorders or injuries can often be multifactorial; aging, trauma, mechanical forces, conformation, hormonal and genetic factors contributing to varying degrees.

Tendinitis is, for a (race) horse also referred to as "incurable disease", one of the most serious musculoskeletal disorders. Tendinitis, rupture of the collagen fibrils, is causing exhibit symptoms such as sudden lameness and swelling. As an example, (partial) tearing of the superficial flexor tendon leads to tendinitis in horses.

Injuries of the suspensory ligament (interosseous muscle) are common in forelimbs and hindlimbs of horses. Lesions are typically classified as affecting the proximal, body, or branches of the suspensory ligament.

Arthritis is multifactorial joint disease, characterized by inflammation, pain and functional impairment. A joint is formed where two bones meet. The healthy joint bones are lined with spongy cartilage which act as shock absorbers, and the synovial fluid, which is secreted by the synovial membrane lining the joint space, acts as a lubricant that prevents friction. Joint strengthening through exercise, weight maintenance, and non-steroidal anti-inflammatory drugs (NSAIDs) can alleviate the symptoms. Osteoarthritis is a multifactorial degenerative joint disease, which progresses over time and results in joint stiffness, inflammation, and pain. Osteoarthritis is hallmarked by progressive degeneration and the wearing away of the cartilage (the cushion between the joints), and typically occurs at the large joints like the hips and knees. It can be a normal age-related degenerative process, occurring gradually after physiological wear and tear.

Joint injuries and conditions as described above afflict numerous mammals, including domesticated animals such as dogs, cats, and horses. In particular, horses often sustain considerable joint injuries due to their participation in farm work, or sporting events, like racing, hunter-jump, dressage, rodeo, barrel-racing, reining and cutting. Lameness due to traumatic joint disease is a common clinical problem in horses and is one of the most important sources of financial losses in the equine industry.

In another or further embodiment of the invention, there is provided a method of treating a wound or inducing tissue regeneration in a subject in need thereof, comprising the administration of a therapeutically effective amount of the collagen formulation as described herein to the subject, thereby treating the wound or inducing tissue regeneration.

The invention is further described by the following non-limiting examples which further illustrate the invention, and are not intended to, nor should they be interpreted to, limit the scope of the invention.

### EXAMPLES

The present invention will now be further exemplified with reference to the following example(s). The present invention is in no way limited to the given examples or to the embodiments presented in the figures.

### Example 1: Cell retention test in a perfusion culture system.

The goal of any cell delivery strategy is to transplant a sufficient number of cells into the region of interest and to achieve maximum retention of cells within that area. Retention may be defined as the fraction of transplanted cells retained in a target tissue or site for a period of time (minutes, hours or weeks). The local milieu is an important determinant of cell retention, as it will influence short-term cell survival.

The cell retention is assessed in a perfusion culture system with the physiological conditions of an equine joint. Five different formulations were generated to assess the impact of the collagen and the ratio of the collagen in the formulation on the cell retention, as visualized in Figure 1. Formulation I comprises soluble collagen and tenogenic induced mesenchymal stem cells in equal amounts. Formulation II comprises soluble collagen, platelet rich plasma and tenogenic induced MSCs in a ratio of 1:1:1. Formulation III comprises soluble collagen, platelet rich plasma and tenogenic induced MSCs in a ratio of 2:1:1. Formulation IV comprises platelet rich plasma and tenogenic induced MSCs in a ratio of 1:1. And formulation V comprises solely tenogenic induced MSCs. In this example the tenogenic induced MSCs are tenogenic induced equine allogeneic peripheral blood-derived mesenchymal stem cells and the platelet rich plasma contains 100 000 000 platelets per ml.

Once the formulations are administered in an individual test system, the amount of cells is counted per system at five different time points, starting with the time of administration, followed by cell counts after 2 hours, 4 hours, 6 hours and 48 hours after the time of administration. Results from the test demonstrate that the combination of collagen, platelet rich plasma and tenogenic induced MSCs shows the highest cell retention of MSCs per ml 2 hours after administration in a 1:1:1 ratio, while the same formulation in a 2:1:1 ratio shows the highest cell retention of MSCs per ml after 48 hours after administration. It is preferred to retain about 100 000 stem cells in the affected tissue after 48 hours after the administration.

### Example 2: Desmitis of the accessory ligament of the equine deep digital flexor tendon

A 4-year-old Trakehner was observed with a sudden onset of lameness and a localized swelling in the proximal metacarpus within 24 hours after a jumping exercise. The distal limb and carpal flexion test were inconclusive. Ultrasonographic examination was performed and confirmed a case of desmitis of the accessory ligament of the deep digital flexor tendon (ALDDFT). The horse was initially treated conservatively for 5 days with non-steroidal anti-inflammatory drugs (NSAIDs), resting, cooling and a compressive bandage in order to reduce the swelling of the site to obtain optimal conditions for the intralesional injection. After conservative treatment, a single intralesional injection of the collagen formulation with autologous tenogenic induced mesenchymal stem cells according to the current invention was assigned with ultrasound guidance. In the days following the intralesional injection, the horse was immobilized for 3 weeks, followed by daily walking exercise for another 3 weeks. Meanwhile particular attention was paid to observing any possible adverse effect or hypersensitivity reaction, local warmth, sweating, heavy breathing or fever. No adverse effects were noted. The horse was clinically sound, the swelling disappeared and ultrasound images at 6 weeks and 4 months after a single intralesional injection showed great tissue repair and negligible scar formation. After positive clinical and ultrasonographical outcome the horse went back to full training without relapse.

### Example 3: Superficial digital flexor tendinitis in a racing Thoroughbred

An adult Thoroughbred presented severe lameness and the involved structures were hot, painful, and swollen. Ultrasonographic examination confirmed a superficial digital flexor tendinitis (SDFT). The collagen formulation suitable for injection was injected into the injured SDFT under ultrasonographic guidance. Treatment of the tendinitis involved a period of rehabilitation with minor inflammatory signs, a period of repair with a significant reduction of inflammatory signs and a final period of remodeling of the tendon, all facilitated by the injected collagen formulation, comprising type I collagen in a neutralizing DMEM solution according to the current invention. Ultrasonography was used as a noninvasive technique for further monitoring of the tendon repair and remodeling. The formation of excessive scar tissue was limited and a normal tendon matrix was regenerated after 12 months of monitoring, enabling the horse to return to performance.

### Example 4: Regenerative therapy for equine degenerative joint disease

A Percheron of over 15 years of age presented a moderate form of clinical lameness for the past 3 months, presumably attributed to fetlock joint osteoarthritis. A positive intra-articular anesthesia of the fetlock joint, and moderate positive flexion test confirmed the location of lameness. Furthermore, osteophytes and cartilage defects were noticed on the CT and radiography, further confirming fetlock joint osteoarthritis. A kit according to the current invention comprising the acidic collagen solution, plasma and chondrogenic induced mesenchymal stem cells was defrosted and the collagen formation was prepared for injection as described in the current invention. The acidic collagen solution and plasma were added together in a 2:1 ratio. Subsequently the chondrogenic induced mesenchymal stem cells were added to the collagen formulation respecting a 1:1 ratio with the plasma and aspirated in the luer lock syringe provided in the kit. The formulation was intraarticularly injected in the horse, followed by daily examination of the injected joint. The horse was closely monitored and showed only minor adverse effects. Further evaluation of the horse during rehabilitation showed an improved functionality and sustainability of the damaged joint in the horse with moderate lameness, due to fetlock joint osteoarthritis.

The present invention is in no way limited to the embodiments described in the examples. On the contrary, methods according to the present invention may be realized in many different ways without departing from the scope of the invention.

## Claims

1. A method for preparing a collagen formulation suitable for injection, comprising the steps:
a) providing a collagen solution having a pH which is lower than 6 ; and
b) adding an amount of liquid neutralizing agent to said collagen solution, resulting in the collagen formulation,
**characterized in that**, said collagen formulation has a pH of between 6.5 and 8.5.

2. Method according to claim 1, **characterized in that**, the pH of the collagen formulation is between 7 and 7.8, preferably 7.4.

3. Method according to claim 1 or 2, **characterized in that**, said neutralizing agent is selected from the group consisting of a plasma, a physiological solution and/or an alkaline solution.

4. Method according to one of the preceding claims 1-3, **characterized in that**, the ratio of the initial collagen solution and the liquid neutralizing agent is between 4:1 to 1:1.

5. Method according to one of the preceding claims 1-4, **characterized in that**, the ratio of the initial collagen solution and the liquid neutralizing agent is 2:1.

6. Method according to one of the preceding claims 1-5, **characterized in that**, the liquid neutralizing agent is plasma, preferably a platelet rich plasma.

7. Method according to claim 6, **characterized in that**, the ratio of the initial collagen solution and plasma is between 4:1 and 1:1.

8. Method according to one of the preceding claims 1-7, **characterized in that**, stem cells are added to the collagen formulation.

9. Method according to claim 8, **characterized in that**, the ratio of the stem cells and the initial collagen solution is between 1:4 and 1:1.

10. Method according to one of the preceding claims 1-9, **characterized in that**, the pH of said collagen solution in step a) is adjusted to a pH between 4.5 and 5.5 with the alkaline solution, prior to adding said neutralizing agent.

11. Method according to one of the preceding claims 1-10, **characterized in that**, the final concentration of the collagen in said formulation suitable for injection is in the range of 3-8 mg/ml.

12. Method according to one of the preceding claims 1-11, **characterized in that**, the collagen formulation suitable for injection is administered by an intravenous, intraarticular, intramuscular, or intralesional injection.

13. Method according to claim 12, **characterized in that**, the time point of administration is within 120 minutes after said collagen formulation suitable for injection is prepared at room temperature.

14. A kit for preparing a collagen formulation suitable for injection comprising a collagen solution, preferably type I collagen, at a pH below 6 and at least one neutralizing agent and optionally stem cells, **characterized in that**, an excess of said collagen solution is provided.

15. Kit according to claim 14, **characterized in that**, the volume ratio of said collagen solution, said neutralizing agent and said stem cells is 4:1:1 to 1:1:1.

16. Kit according to claim 14 or 15 or collagen formulation with a pH between 6.5 and 8.5 for use in the treatment of a pathology in the musculoskeletal system in a subject.
